Europäisches Patentamt

European Patent Office    (11) Publication number:    **0 175 421**
                                                    A2
Office européen des brevets

(19)

(12)    **EUROPEAN PATENT APPLICATION**

(21) Application number: **85201467.9**    (51) Int. Cl.⁴: **C 12 Q 1/14,** C 12 Q 1/00

(22) Date of filing: **16.09.85**

(30) Priority: **17.09.84 US 651383**    (71) Applicant: **WARNER-LAMBERT COMPANY, 201 Tabor Road, Morris Plains New Jersey 07950 (US)**

(43) Date of publication of application: **26.03.86 Bulletin 86/13**    (72) Inventor: **Buenviaje Wantland, Nora M., 21, Egbert Hill, Morristown N.J. 07960 (US)**
Inventor: **Seo, John S., Rri Box 122, Augusta N.J. 07822 (US)**

(84) Designated Contracting States: **BE CH DE FR GB IT LI NL SE**    (74) Representative: **Douma, Anno Dominicus et ai, Postbus 20, NL-5340 BH Oss (NL)**

(54) Test method for the presumptive identification of streptococci.

(57)    The identification of streptococci can be carried out simply and quickly using a series of tests and comparison of the test results with an identification scheme.

EP 0 175 421 A2

1

# TEST METHOD FOR THE PRESUMPTIVE IDENTIFICATION OF STREPTOCOCCI

This invention relates to a method for identifying bacteria. In particular it relates to a method for identifying streptococci commonly isolated from humans, by utilizing the ability of enzymes produced by the streptococci to hydrolyze certain compounds, the hydrolysis products being detected by use of specific reagents.

Bacterial diseases are diagnosed and treated by the isolation and identification of causative microorganisms. Strictly speaking, medical therapy should be initiated only after determination of the etiologic agent. Clinical tests for bacterial identification depend upon the comparison of a number of physiological, morphological and positive and/or negative biochemical reactions between known and unknown species. To accomplish this task, it is necessary to obtain specimen cultures of the organism from such sources as sputum, blood, urine, etc., and to submit these samples to identifying procedures.

Streptococci are one of the most ubiquitous bacteria in nature. They are responsible for numerous illnesses including streptococcal pharyngitis, scarlet fever, puerperal sepsis, impetigo or cellulitis, rheumatic fever, acute glomerulonephritis and subacute bacterial
endocarditis. Other streptococcal infections include streptococcal
sore throat, erysipelas and septicemia.

Streptococci commonly isolated from humans include alpha and
beta-hemolytic streptococci, namely groups A, B, D enterococcus;
D non-enterococcus, Viridans group and Streptococcus pneumoniae.
Conventional means of identification require time consuming techniques
which are concomitantly prone to possible error and mis-
identification. Facklam et al. teach a method for the identification
of streptococci utilizing L-pyrrolidonyl-B-naphthylamide, based on the
hydrolysis of the naphthylamide, in conjunction with CAMP and bile-
esculin tests, to presumptively identify the streptococci. The tests
purportedly correctly identified 98% of 50 group A; 98% of 46 group B;
and 100% of 70 strains that were not group A, B or D. See "Presumptive
Identification of Streptococci with a New Test System" R.R. Facklam et
al., Journal of Clinical Microbiology 1982 pp. 987-990. The test
procedure, however, requires a 16- to 20-hour incubation periods.

Conventional test methods for the identification of streptococci
include bacitracin, "CAMP" factor, esculin, and growth in 6.5% NaCl.
Extraction and precipitation tests are also used. Carbohydrate
antigens are extracted by boiling the organisms in dilute acid at
100 °C for 1 hour and reacting the antigen with commercially prepared
group specific antiserum. Additionally, fluorescent antibody
techniques can be used. These methods require a variety of individual
tedious procedures. Often identification is not obtained until the
third to fourth day after a clinical specimen is received. As a result
the information obtained becomes essentially useless in cases of
critical or life threatening infections.

Various methods and apparatus have been employed in an attempt to
facilitate the identification of microorganisms. These are primarily
directed to expediting the cumbersome process and rendering the
identification more positive. To date, a most successful advance in
this area of clinical laboratory testing involves the use of bibulous
paper or other absorbent substrates or matrices impregnated with reagents which detect the presence of specific enzymes or metabolic end products characteristic of certain microorganisms. These reagents include a substrate to be acted upon by a specific bacterial enzyme and a detection system which reacts with the metabolic end product to yield a readily identifiable color change. U.S. Patent Nos. 3,122,480; 3,341,427; 3,359,180; 3,378,346; 3,597,321; 3,616,258; 3,645,853 and 3,649,461 disclose the preparation and formulation of various substrates and detection reagents as well as their application to the identification of certain organisms. U.S. Patent No. 4,260,687 discloses an apparatus which can be utilized to simplify such tests.

More recently a test procedure and kit system identified as API-20 Streptococcus (Analytab Products) has been developed. While an incubation period of only 3 to 4 hours is required, some twenty tests must be performed. Although the tests are rapid, the kit, which includes a tray, is expensive and good for only one identification. Additionally, it is difficult to use and some reactions work poorly or are not clear cut.

This test system utilizes various substrates in conjunction with particular reagents. Included among the twenty tests are:

| Substrate | Reagent |
|---|---|
| L-pyrrilidonyl-β-naphthyl-amide (PYR) | 4-dimethyl-aminocinn-amaldehyde |
| Ortho-nitrophenyl-β-D-galactoside (ONPG) | Ortho-nitrophenol (yellow end product) |
| Sodium hippurate (HIP) | Ninhydrin |
| Bile-esculin (BEM) | Ferric citrate |

The bile esculin and ONPG tests are described in Biochemical Tests For Identification of Medical Bacteria, Jean F. MacFaddin, William and Wilkins, Baltimore 1976. Hence the individual tests and their method of use are old in the art. What is required is a simplified method for streptococcal identification which can be conducted at low cost and at the same time result in identification in which an investigator can have a high level of confidence.

0175421

It has surprisingly been found that streptococci can be identified accurately and quickly by utilizing four known bacterial identification tests together with hemolysis tests. The tests include PYR, ONPG, esculin and sodium hippurate which involve using the foregoing substrates to be acted upon by the bacteria to be identified, determining whether the test is positive (+) or negative (-), and determining the type of hemolysis, if any, which occurs.

A solution of substrate and nutrients is used to impregnate a paper strip which is preferably placed in a carrier having at least one well for the deposition of an aliquot of test organism. After incubation for 4 hours at 35 to 37 °C, a reagent is added, if necessary, in order to determine whether the test results are positive or negative.

Where the substrate is a hippurate, for example sodium hippurate, the reagent is ninhydrin. A color of lavender to blue indicates a positive reaction and the substantial absence of reaction is indicated by the failure to develop color.

Where the substrate is PYR, the reagent is 4-dimethylamino-cinnamaldehyde. A positive result, i.e. reaction, is indicated by a pink to purple color, usually red to red-orange, and a negative result is indicated by yellow or light orange.

Where esculin is the substrate, the reagent is ferric citrate and a positive result is indicated by a black color. In the case of ONPG, no reagent is necessary since hydrolysis of the ONPG results in the formation of ortho-nitrophenol, which has a yellow color.

Hemolysis is carried out using red blood cells, for example sheep blood cells. The test specimen is cultured with the blood cells in agar. In the case of beta hemolysis a clear ring appears around the colony, while with alpha hemolysis the cells take on a green color.

The results of the tests are recorded and, by using an identification chart such as that shown in Table I below, the particular streptococci can be grouped with a high degree of accuracy.

This invention relates to a method for identifying streptococci. In particular it relates to a simple, rapid technique which can be utilized to correctly group streptococci using a minimum of apparatus and analytical technique.

In the practice of this invention known tests are used together with hemolysis to group the streptococci. The four tests are the L-pyrrolidonyl-β-naphthylamide, sodium hippurate, ortho-nitrophenyl-β-D-galactoside and esculin tests.

The invention involves in its broadest aspects, a method and kit for identifying streptococci. The method comprises:

a) preparing a suspension of a streptococci specimen to be identified;

b) preparing test elements for ortho-nitrophenyl-β-D-galactoside, L-pyrrolidonyl-β-naphthylamide, hippurate, and ferric citrate incorporated esculin tests, each test element comprising a substrate and a nutrient or buffer;

c) applying an aliquot of specimen suspension to each test element and incubating the test elements so treated for about 3 to about 4 hours at about 35° to about 37 °C;

d) applying a ninhydrin reagent to the treated incubated hippurate test element, applying a paradimethylaminocinnamaldehyde reagent to the treated incubated L-pyrrolidonyl-β-naphthylamide test element, incorporating esculin and further incubating the test elements;

e) evuluating the test to determine whether a positive or negative reaction occurred;

f) determining whether alpha, beta, or no hemolysis occurred;

g) comparing the L-pyrrolidonyl-β-naphthylamide, hippurate, esculin, ortho-nitrophenyl-β-D-galactoside and hemolysis test results with a suitable identification scheme chart; and

h) making a presumptive identification of the test specimen based on said comparison.

The kit comprises: at least one test element for each of an esculin, L-pyrrolidonyl-β-naphthylamide, ortho-nitrophenyl-β-D-galactoside and hippurate test; a reagent for each of such tests, and an identification scheme chart.

Each of the tests is conducted in the same manner. A substrate, the compound from which the test takes its name, is dissolved in a nutrient broth to which a culture of the specimen to be identified is added. The enzymes produced by the bacteria act on the substrate to produce a compound which is detected either directly or by the addition of a reagent.

In the ortho-nitrophenyl-β-D-galactoside (ONPG) test, the enzyme β-galactosidase produced by the Streptococcus pneumoniae will act on the ONPG to cleave it by hydrolysis. In the process, ortho-nitrophenol, which has a yellow color, is produced. The change from colorless to yellow is a positive indication of the existence of Streptococcus pneumoniae.

The esculin test (BEM) is a measure of the ability of an organism to hydrolyze the glycoside esculin to esculetin and glucose. Esculetin (6,7-dihydroxycoumarin) will react with iron salts to form a black precipitate. The production of a black color in the presence of iron salts, for example ferric citrate, is a positive reaction.

However, since both group D enterococcal and D non-enterococcal streptococci will give this positive reaction, the test must be used in conjunction with other tests to identify enterococci. For example, some species of Viridans S. sanguis, S. mutans and S. anginosus are capable of splitting esculin. However, these species cannot tolerate concentrations of bile of 40% or more. Hence, it is preferred that bile be included in the test medium at concentrations of about 40% by weight or more.

Alternatively, sodium azide may be used as an inhibitor for Viridans strains; however, a medium containing a high concentration of bile (40%) and no azide is better for differentiation. A suitable bile esculin strip is marketed under the trademark Pathotec[R] by Warner-Lambert Company's General Diagnostic Division.

In the sodium hippurate test the bacteria hydrolyze hippurate to produce benzoic acid and glycine. The glycine is detected by the addition of ninhydrin which produces a lavender to blue color for a positive test. No color is generated for a negative test. Only group B streptococci give a positive hippurate test.

In the L-pyrrolidonyl-β-naphthylamide test the substrate is hydrolyzed to release β-naphthylamine which reacts with 4-dimethylamino-cinnamaldehyde to produce a red to red orange color (positive). Light orange, yellow, or no color represents a negative result.

Bacteria are classified as being alpha hemolytic or beta hemolytic. Hemolysis is determined by preparing an agar test plate containing beef or heart infusion as a nutrient. The test plates are prepared by streaking or otherwise applying the specimen containing the bacteria to be tested to a blood agar test plate and culturing via incubation at about 35° to about 37 °C for about 18 hours. Beta hemolytic bacteria cause complete lysis of the blood cells and the development of a clearing area around the colony. The beta hemolysis is readily observed by holding up the test plate, which was initially opaque, to light. After the test, transparent rings surround each colony of bacteria indicating a positive result.

Other conventional hemolysis testing techniques can be used, in combination with or instead of, this test.

In the case of alpha hemolytic bacteria the colonies have a green appearance resulting from conversion of oxyhemoglobin to methemoglobin. Some bacteria will exhibit neither alpha nor beta hemolysis, and the test plate remains substantially unchanged.

The tests of this invention will generally be carried out in the liquid phase. However, improved results have been achieved by preparing test discs of a bibulous paper impregnated with the substrate and a nutrient broth. The dried test discs can then be used in any container adapted to hold the disc. While any container such as a test tube may be used it is preferred that an apparatus such as that disclosed in U.S. Patent No. 4,260,287 be used.

8  0175421

Any bibulous material can be used to form the test discs provided that it is an absorbent material which is substantially free of impurities which may interfere with the tests to be performed. Illustrative non-limiting examples of such bibulous materials are Whatman #1 filter paper and chromatography paper. While the test elements are referred to as discs, it will be evident to those skilled in the art that the shape of the test element is not critical and any shape is acceptable. The discs, however, are a more convenient shape for use with the apparatus of U.S. Patent No. 4,260,287.

The substrate and nutrients can be coated onto the bibulous material by suitable methods, e.g., by roller coating from a solution or by immersion of the material in the solution. The bibulous material must contain at least an effective amount of substrate. Impregnation of the bibulous material with substrate solutions known in the art, such as those taught in Biochemical Tests For Identification of Medical Bacteria, supra, provides an effective amount of substrate and nutrients on the material. Where roller coating is to be used, the concentration of the substrate in coating solution should be about at least twice the level used for conventional liquid phase tests. Preferably, coating is accomplished by dipping a rotating metal wheel into the solution of substrate and nutrients and passing the bibulous material between the wheel and a nip roll.

The inventors do not claim the aforedescribed tests, per se, as their invention. The tests, which are designated in the art by the name of the substrate used, are well known, as is the method of preparing solutions of substrates and nutrients for each test.

The invention here arises from the discovery that the four tests described - that is, sodium hippurate (HIP); L-pyrrolidonyl-β-naphthylamide (PYR); ortho-nitrophenyl β-D-galactoside (ONPG); and esculin - when used in conjunction with blood hemolysis determinations, followed by evaluation of the test results using the Identification Scheme of following Table I or a similar scheme, will permit the accurate identification of streptococci.

0175421

TABLE I

STREPTOCOCCI IDENTIFICATION SCHEME

Streptococci

| Test method | A | B | Not A, B or D | D Entero-coccus | D non-Entero-coccus | Viridans | Strepto-coccus pneumoniae |
|---|---|---|---|---|---|---|---|
| Hemolysis | Beta | Beta | Beta | Beta, alpha or none | Beta, alpha or none | Alpha | Alpha |
| PYR | + | − | − | + | − | − | − |
| Sodium hippurate | − | + | − | − | − | − | − |
| ONPG | − | − | − | − | − | − | + |
| Esculin | − | − | − | + | + | − | − |

(+) indicates positive test results

(−) indicates negative test results


It should be noted that para-nitrophenyl-$\beta$-D-galactoside may be substituted for the ortho compound. The nitrophenol which is released during hydrolysis is colorless in acid solution. However, it undergoes a tautomeric change in alkaline solution producing a yellow color. Hence, when using the para compounds, the indicator system must include a buffer to maintain alkalinity.

It will be appreciated that when using a bibulous material in carrying out the tests of this invention a wide range of concentrations of substrate and nutrients in solution can be used. These concentrations will depend upon the method of application of the solutions to the bibulous material. While non-deleterious excesses can be tolerated, only an effective amount of the compositions need be associated with the dried bibulous material. Such amounts are readily determined by using known bacterial cultures and testing the results obtained at various solution concentrations and methods of application. An "effective amount" is the amount necessary to give a correct positive reading with sufficient clarity to minimize operator error, and will depend, in part, on the desired intensity of color selected as acceptable.

The following examples serve only to illustrate a suitable manner of preparing the bibulous material of this invention and are not intended to limit the invention.

## Example I
### HIPPURATE TEST

Substrate Solution

A solution containing the substrate sodium hippurate and nutrients was prepared and applied to chromatography paper. The solution formulation concentration is as follows:

| | |
|---|---|
| Sodium Hippurate | 25.0 g |
| Proteose Peptone #3 | 11.0 g |
| Yeast Extract | 1.0 g |
| Dextrose | 1.26 g |
| Distilled Water | Q.S. to 100 ml |

The pH of the solution was adjusted to about 7.2 with 1 NaOH. The solution was applied to the paper by roller coating onto one side only. ¼ inch discs were punched out of the dried paper for use as test elements.

In conducting the hippurate test with other tests of this invention a standard reproducible bacterial suspension should be used. A suspension of test organism in physiological saline having an optical density of MacFarland #0.5 is adequate.

Reagent Solution

The reagent for the hippurate test is ninhydrin. A reagent solution comprising 3.5 grams of ninhydrin in 100 ml of a 1:1 acetone/butanol mixture is an acceptable reagent solution.

11  0175421

In conducting the hippurate test a 0.2 ml aliquot of organism suspension is applied to the test element in a suitable container and incubated for about 4 hours at 35° – 37 °C. Two drops of the reagent solution is then added with incubation for an additional ten minutes and the treated element is observed for color reaction. A lavender to blue color indicates a positive test. No color indicates a negative response.

## Example II
### PYR TEST

Test elements were prepared for the PYR test in the same manner as in Example I except

Substrate Solution

| | | |
|---|---|---|
| L-pyrrolidonyl-β-naphthylamide | 0.30 | g |
| Ethanol (95%) | 50 | ml |
| Distilled water | 50 | ml |
| Proteose Peptone #3 | 22.0 | g |
| Yeast extract | 4.0 | g |
| Dextrose | 4.0 | g |

Reagent Solution

| | | |
|---|---|---|
| 4-dimethylaminocinnamaldehyde | 0.15 | g |
| 2-methoxyethanol | 50 | ml |
| Glacial acetic acid | 25 | ml |
| Sodium dodecyl sulfate | 25 | g |
| Distilled $H_2O$ | 925 | ml |

The test is carried out in the same manner as Example I. A pink to purple color range is a positive indication; while yellow to light orange is negative.

## Example III

### ONPG TEST

Test elements were prepared in the same manner as Example II except that the substrate solution was applied to one side of the paper and a buffer solution to the other side.

Substrate Solution

| | |
|---|---|
| Ortho-nitrophenyl-β-D-galactoside | 150.0 g |
| Acetone | 2.1 l |
| Distilled water | 0.9 l |

Buffer Solution

| | |
|---|---|
| Potassium phosphate monobasic | 20.06 g |
| Sodium phosphate dibasic | 121.06 g |
| Distilled water | Q.S. to 1 liter |

No nutrient broth is required for the ONPG test. The buffer solution must have sufficient basicity so that a 1:1 mixture of substrate solution with buffer solution has a pH of at least 7.0.

The test is conducted in the same manner as Example II. No reagent is required since any nitrophenol formed has a yellow color in an alkaline solution. A yellow color is positive. A colorless or light yellow product indicates a negative response.

## Example IV

### ESCULIN TEST

Test elements for the esculin test are prepared in the same manner as Example II.

Substrate Solution

| | |
|---|---|
| Klucel | 3.0 g |
| Methanol | 0.93 l |
| Esculin | 30.9 g |
| Distilled water | 0.6 l |

Nutrient Broth

| | | |
|---|---|---|
| Heart Infusion Broth | 200 | g |
| Ferric Citrate | 80 | g |
| NaOH, 1N | Q.S. for pH 7.0 | |
| Distilled water | Q.S. to 1 liter | |

In this case the nutrient broth includes the reagent ferric citrate. Klucel is used as a film former to prevent dusting or flaking of esculin which is a powdery non-film former.

The esculin test is run in the same manner as Example III except that since the reagent is already included on the test element a direct reading is obtained. A black or dark brown color indicates a positive reaction. A tan or light gray color is a negative result.

A dry test system can be prepared without the use of a bibulous material. The appropriate solutions need only be added to test wells and evaporated to dryness. For example, the solutions of Example IV are diluted by diluting 7.5 ml of solution to 100 ml with distilled water. A 0.1 ml aliquot of substrate solution is added to the test well and evaporated to dryness. Then a 0.1 ml aliquot of nutrient broth is diluted by diluting 7.5 ml of solution to 100 ml with distilled water. A 0.1 ml aliquot of substrate solution is added to the test well and evaporated to dryness. Then a 0.1 ml aliquot of nutrient broth is added to the test well and evaporated to dryness. The test is performed in the same manner as Example III without the need for any bibulous material. Similar test wells can be prepared in the same manner for each of the other tests. The test well can comprise a test tube, the apparatus of U.S. Patent No. 4,260,687, or any suitable container of approximately ¼ inch diamter. The size of the test well is not critical. However, ¼ inch diameter is a convenient size, requiring a minimum amount of material while giving good results.

14          0175421

As used in the specification and claims the term "test element" means either a bibulous material having been treated in accordance with the aforegoing description or the test wells containing the dried substrate and nutrient or buffer solutions. Generally, when test wells are to be prepared containing the dried reactants each of the aforedescribed solutions can be utilized by diluting 7.5 ml of solution to 100 ml with distilled water, adding 0.1 ml aliquot to the well and evaporating to dryness. However, when ONPG is used, it is diluted 1:1 with distilled water.

### Example V

Test elements were prepared in the manner of Example I for the hippurate, PYR, ONPG and esculin tests using the formulations set forth in Examples I - through IV, respectively. Additionally, dry test elements without bibulous materials were prepared using the apparatus described in U.S. Patent No. 4,260,687 as the test wells.

For the 73 strains included in the study there was 100% agreement between identification obtained by the test method of this invention for both the discs and the dried test elements to conventional identification methodology. In general, the dry test elements gave better color reaction than the disc test elements.

By comparison using the API 20-S tests forty-one of the total strains were correctly identified. Thirty strains produced octal codes which were not listed in the API 20-S Analytical Profile Index. Two strains of Group A were misidentified as non-group A, B or D.

Table II summarizes the data obtained using the API 20-S test kit.

0175421

TABLE II

Results of Testing 73 Strains of Streptococci on API 20-S

| Group/Species | Total | Correctly Identified | Not Identified[1] | Misidentified |
|---|---|---|---|---|
| A | 18 | 10 | 6 | 2[2] |
| B | 12 | 11 | 1 | |
| D enterococcus | 20 | 6 | 14 | |
| D nonenteroccus | 3 | 2 | 1 | |
| Streptococcus pneumoniae | 13 | 11 | 2 | |
| Viridans Group | 4 | | 4 | |
| Not A, B or D | 3 | 1 | 2 | — |
| TOTAL | 73 | 41 | 30 | 2 |

1 - Octal code non-existent in ID Manual.

2 - Misidentified as not A, B or D.

As used in the specification and claims the terms "PYR test", "ONPG test", "hippurate test" and "esculin test" mean the respective streptococci tests known in the art which take their names from the substrates L-pyrrolidonyl-β-naphthylamide, ortho- or para-nitrophenyl-β-D-galactoside, alkali metal, e.g., sodium hippurate and esculin, respectively used in the tests. The ONPG substrate is preferably ortho-nitrophenyl-β-D-galactoside. The alkali metal hippurate is preferably sodium hippurate.

As used in the specification and claims the term "identification scheme" means the scheme of streptococci identification which utilizes the PYR, ONPG, hippurate and esculin tests in conjunction with hemolysis tests, the results of which tests are evaluated using the Figure. As used in the specification and claims the term

16   0175421

"identification scheme chart" and "identification scheme" refer to Table I of this specification and/or other suitable diagrams by which suitable test results can be used to identify streptococci.

Reasonable variations, such as those which would occur to a skilled artisan, can be made herein without departing from the scope of the invention.

0175421

1

## CLAIMS

1.    A method for the presumptive identification of streptococci
which comprises:

a) preparing a suspension of a streptococci specimen to be
identified;

b) preparing test elements for ortho-nitrophenyl-β-D-galactoside,
L-pyrrolidonyl-β-naphthylamide, hippurate, and ferric citrate
incorporated esculin tests, each test element comprising a
substrate and a nutrient or buffer;

c) applying an aliquot of specimen suspension to each test element
and incubating the test elements so treated for about 3 to 4
hours at about 35° to about 37 °C;

2    0175421

d) applying a ninhydrin reagent to the treated incubated hippurate test element, applying a para-dimethylaminocinnamaldehyde reagent to the treated incubated L-pyrrolidonyl-β-naphthylamide test element, incorporating esculin, and further incubating the test elements;

e) evaluating the test to determine whether a positive or negative reaction occurred;

f) determining whether alpha-, beta-, or no hemolysis occurred;

g) comparing the L-pyrrolidonyl-β-naphthylamide, hippurate, esculin, ortho-nitrophenyl-beta-D-galactoside and hemolysis test results with a suitable identification scheme chart; and

h) making a presumptive identification of the test specimen based on said comparison.

2.    The method according to claim 1 wherein the hemolysis determination is carried out using colonies prepared by applying the specimen to a blood agar test plate and incubating the test plate at about 35° to about 37 °C for about 18 hours.

3.    The method according to claim 1 wherein the reagent treated test elements are further incubated for about 10 minutes.

4.    The method according to claim 1 wherein the substrate of the esculin test includes bile.

5.    The method according to claim 4 wherein the bile is present at about 40% by weight based on the esculin.

6.    The method according to claim 1 wherein the test elements comprises a bibulous material impregnated with the substrate materials and a nutrient or buffer.

7.    The method according to claim 7 wherein the bibulous material is paper.

8.    The method according to claim 1 wherein the test elements comprise test wells which have been prepared by depositing there into respective substrates and nutrients or buffers and evaporating solvent solutions of the solvent, thereby leaving dried substrates and nutrients or buffers in each of the respective test wells.

9.      A streptococci identification kit comprising at least one test element for each of an esculin, L-pyrrolidonyl-β-napththylamide, ortho-nitrophenyl-β-D-galactoside and hippurate test; a reagent for each of such tests, and an identification scheme chart.